(19) Europäisches Patentamt
European Patent Office
Office européen des brevets

(11) Numéro de publication : **0 121 466**
**B1**

(12) # FASCICULE DE BREVET EUROPÉEN

(45) Date de publication du fascicule du brevet : **19.08.87**

(51) Int. Cl.[4] : **C 07 C 46/00**, C 07 C 50/18

(21) Numéro de dépôt : **84400567.8**

(22) Date de dépôt : **21.03.84**

(54) **Procédé de décomposition d'un complexe d'acide orthobenzoyl-benzoique, de fluorure d'hydrogène et de trifluorure de bore.**

(30) Priorité : **30.03.83 FR 8305207**

(43) Date de publication de la demande : **10.10.84 Bulletin 84/41**

(45) Mention de la délivrance du brevet : **19.08.87 Bulletin 87/34**

(84) Etats contractants désignés : **BE CH DE FR GB IT LI NL SE**

(56) Documents cités :
**EP-A- 0 055 951**
**US-A- 1 895 788**
**US-A- 2 496 894**
**CHEMICAL ABSTRACTS, vol. 94, 1981, page 414, no. 15446q, Columbus, Ohio, US**

(73) Titulaire : **ATOCHEM**
**4 & 8, Cours Michelet La Défense 10**
**F-92800 Puteaux (FR)**

(72) Inventeur : **Devic, Michel**
**27 Chemin des Fonds**
**F-69110 Sainte-Foy-lès-Lyon (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

EP 0 121 466 B1

**Description**

La présente invention concerne un procédé de décomposition de complexes formés d'acide ortho-benzoyl-benzoïque (acide OBB), de fluorure d'hydrogène (HF) et de trifluorure de bore ($BF_3$), de formule générale :

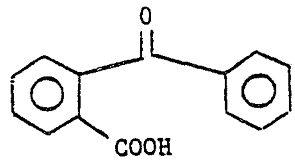 , n HF, $mBF_3$ (I)

dans laquelle n et m sont compris entre 1 et 6.

De tels complexes peuvent être obtenus selon la demande de brevet européen publié sous le n° 0 055 951 au nom de la demanderesse.

Les brevets US 1 895 788 et US 2 496 894 décrivent des procédés de cyclisation de l'acide OBB en utilisant de l'acide sulfurique concentré ou de l'oléum. La demande de brevet japonais n° 78/120116 du 29 septembre 1978 décrit la cyclisation de certains acides OBB substitués par des alkyles à l'aide d'oléum et d'acide borique. Tous ces procédés utilisent comme réactif de départ un acide OBB isolé et non pas un complexe acide OBB, HF, $BF_3$.

Afin de pouvoir isoler l'acide OBB contenu dans de tels complexes, en vue essentiellement de sa cyclisation en anthraquinone, ceux-ci doivent subir par exemple un traitement par de l'eau bouillante ou par de la soude. Un traitement de cette nature a l'inconvénient majeur de provoquer la destruction de l'HF et du $BF_3$ et par conséquent de rendre non économiquement viable la récupération du catalyseur HF, $BF_3$ utilisé dans la synthèse de l'anthraquinone décrite dans la demande de brevet européen citée plus haut.

Un chauffage prolongé du complexe à 150 °C-200 °C permet de le décomposer mais entraîne simultanément la dégradation presque totale de l'acide OBB lui-même. L'eau formée au cours de cette dégradation se combine de plus à l'HF et au $BF_3$ en rendant ainsi le catalyseur impropre à un recyclage direct.

Un chauffage prolongé sous vide du complexe à une température plus basse, par exemple 50 °C à 100 °C, permet de limiter la décomposition de l'acide OBB mais laisse subsister une partie du catalyseur dans cet acide OBB, ce qui rend le procédé non économique.

Le chauffage du complexe à faible température dans un solvant inerte n'aboutit encore qu'à une récupération incomplète du catalyseur et provoque une dégradation toujours importante de l'acide OBB.

Dans la demande de brevet français n° 82-14920 au nom de la demanderesse, est décrit un procédé de décomposition du complexe dans lequel celui-ci est soumis à l'action d'un solvant inerte, à une température d'au moins 20 °C, dans une colonne à distiller fonctionnant avec un fort reflux de solvant. Un tel procédé permet de récupérer pratiquement quantitativement l'HF et le $BF_3$ mais présente l'inconvénient de ne permettre d'isoler l'acide OBB qu'après sa séparation d'avec le solvant à l'action duquel est soumis le complexe.

Le procédé de la présente invention permet de pallier les désavantages des méthodes connues. Selon un tel procédé, d'une part la récupération de l'HF et du $BF_3$ est pratiquement quantitative et, d'autre part, l'acide OBB ne subit pratiquement pas de dégradation et peut être, simultanément à la décomposition du complexe, cyclisé en anthraquinone sans avoir à être isolé du milieu dans lequel il se trouve libéré et ce dans les conditions mêmes adoptées pour effectuer la décomposition du complexe.

Dans le procédé de l'invention le complexe de formule générale (I) est soumis, à pression atmosphérique ou à une pression inférieure à la pression atmosphérique, à l'action de l'acide sulfurique de concentration au moins égale à 96 % en poids, ou à celle d'un oléum.

A titre d'exemple la quantité d'acide sulfurique de concentration égale à 96 % en poids est de préférence comprise entre 1 et 3 parties en poids par partie d'acide OBB dans le complexe, celle d'oléum à 20 % en poids de $SO_3$ de 0,5 à 2 parties en poids par partie d'acide OBB.

La température à laquelle s'effectuent simultanément la décomposition du complexe et la cyclisation en anthraquinone de l'acide OBB libéré est le plus souvent comprise entre 100 °C et 180 °C, de préférence entre 120 °C et 150 °C.

Il est particulièrement avantageux, pour faciliter la récupération de l'HF et du $BF_3$, d'opérer, dans une telle gamme de températures, à une pression telle qu'il ne sera pas constaté de départ d'eau du milieu réactionnel. On opérera par exemple de préférence à une pression comprise entre la pression atmosphérique et $4 \cdot 10^{-2}$ bar.

La durée de l'opération, dans le cadre des autres conditions définies ci-dessus, est généralement comprise entre 15 minutes et 2 heures.

Lorsque la réaction est effectuée à une pression inférieure à la pression atmosphérique, l'emploi de l'acide sulfurique est préféré, la température est choisie de préférence égale à 150 °C ou peu différente de cette valeur et la durée de l'opération est de 30 minutes environ.

Lorsque la réaction est effectuée à pression atmosphérique ou à une pression inférieure mais proche de cette valeur, l'emploi d'un oléum, par exemple l'oléum à 20 % en poids de $SO_3$, est préféré à celui de l'acide sulfurique. La température est alors de 120 °C environ et la durée de l'opération de l'ordre de 1 heure.

Au cours de l'opération de décomposition du complexe, l'HF et le $BF_3$ se dégagent à l'état gazeux. L'HF peut être séparé du $BF_3$ par exemple par condensation. L'HF et le $BF_3$, ce dernier après lavage sulfurique à l'aide par exemple de l'acide sulfurique ou de l'oléum mis en jeu pour la décomposition du complexe, peuvent ainsi être réemployés pour constituer le catalyseur HF, $BF_3$ de synthèse de ce même complexe.

Dans une variante particulièrement avantageuse du procédé, l'HF libéré au cours de l'opération de décomposition du complexe peut être partiellement ou totalement transformé en $BF_3$, au fur et à mesure de son apparition, par réaction avec de l'acide borique $H_3BO_3$ introduit par exemple en mélange avec l'oléum ou l'acide sulfurique.

L'anthraquinone formée durant l'opération de décomposition du complexe peut être facilement et économiquement recueillie par exemple en provoquant son insolubilisation par dilution du milieu sulfurique final et en isolant et en purifiant l'anthraquinone ainsi insolubilisée par de simples opérations de filtration et de lavage. La dilution du milieu réactionnel sulfurique final peut être réalisée grâce à une addition d'eau ou, plus judicieusement, grâce à une solution aqueuse d'acide sulfurique, de concentration par exemple égale ou voisine de 70 % en poids, de telle façon que la concentration en acide sulfurique dans le milieu contenant l'anthraquinone atteigne environ 80 % en poids. Les jus acides de filtration peuvent être reconcentrés pour être recyclés dans l'opération de décomposition du complexe ou peuvent servir à la formation de la solution acide mise en œuvre pour diluer la solution sulfurique d'anthraquinone.

Le procédé selon l'invention peut être réalisé en continu ou en discontinu, dans un ou plusieurs réacteurs en série, agités.

La planche unique montre le schéma de procédé correspondant à un mode de réalisation de l'invention en continu, dans deux réacteurs agités en série : le complexe acide OBB, HF, $BF_3$, de formule générale (I), est introduit par la tuyauterie 1 dans le réacteur 2. L'acide sulfurique concentré, ou l'oléum, amenés par la tuyauterie 3 traversent la colonne de lavage 4 avant d'entrer dans le réacteur 2 par la conduite 5. L'HF et le $BF_3$ sortent du réacteur 2 par la tuyauterie 6. La solution sulfurique présente dans le réacteur 2 sort de ce dernier par la conduite 7 et pénètre dans le réacteur 8. L'HF et le $BF_3$ gazeux sortent du réacteur 8 par la tuyauterie 9 et sont réunis à l'HF et au $BF_3$ circulant dans la conduite 6. Le mélange gazeux ainsi formé entre par la tuyauterie 10 en 11 où est réalisée la séparation de l'HF du $BF_3$ par condensation de l'HF. L'HF condensé est évacué par la conduite 12. Le $BF_3$ gazeux entre ensuite, par la conduite 13, dans la colonne de lavage 4 d'où il sort par la tuyauterie d'évacuation du $BF_3$14. La solution sulfurique extraite du réacteur 8 est évacuée par la tuyauterie 15 en vue par exemple des opérations de dilution, de filtration et de lavage décrites plus haut et pratiquées dans des appareillages non schématisés dans la planche unique.

Les exemples suivants, donnés à titre non limitatif, illustrent l'invention selon la variante dans laquelle l'HF libéré du complexe est transformé quantitativement en $BF_3$ par réaction avec l'acide borique $HF_3BO_3$.

## Exemple 1

En procédant selon la demande de brevet européen publiée sous le numéro 0 055 951, on fait réagir, en proportion, 1 mole d'anhydride phtalique et 1 mole de benzène dans 10 moles de HF et 10 moles de $BF_3$. Après dégazage à —40 °C d'une partie du $BF_3$ on obtient, pour 1 mole d'anhydride phtalique engagée, une solution de complexe de l'acide OBB dans HF contenant 203,3 g d'acide OBB, 306,7 g de $BF_3$, 200 g d'HF total et 22,7 g d'impuretés de la réaction, que l'on évapore sous vide à 0 °C pour obtenir, à partir de 100 g de solution initiale, 60 g de solution finale.

Dans un réacteur agité en acier inoxydable on ajoute à ces 60 g de solution, 31,5 g d'acide sulfurique à 96 % en poids et 8,5 g d'acide borique $H_3BO_3$ avant de chauffer alors, sous une pression de $4 \cdot 10^{-2}$ bar, à 150 °C et de maintenir le mélange à cette température durant 20 minutes.

De l'évaluation de la quantité de $BF_3$ sortant du réacteur durant l'opération, on conclut à un rendement de récupération de l'HF et du $BF_3$ initialement contenus dans le complexe, de plus de 99 %, montrant une efficacité de décomposition du complexe pratiquement quantitative.

Après refroidissement, insolubilisation de l'anthraquinone produite à partir de l'acide OBB libéré du complexe par dilution du milieu sulfurique à l'eau, séparation par filtration et lavage de l'anthraquinone ainsi insolubilisée, on obtient 23,6 g d'anthraquinone pure, ce qui correspond à une récupération de l'acide OBB égale au moins à 91,2 %.

## Exemple 2

En traitant, dans le même appareillage et selon le même processus que dans l'exemple 1, la même quantité de la même solution évaporée de complexe que dans l'exemple 1, par 31,5 g d'oléum à 20 % de

$SO_3$, à 120 °C, à pression atmosphérique et durant 30 minutes en présence de 8,5 g d'acide borique, le rendement de récupération de l'HF et du $BF_3$ contenus initialement dans le complexe est encore supérieur à 99 % montrant donc une efficacité de décomposition pratiquement quantitative. Le rendement de récupération de l'acide OBB, calculé à partir de la quantité d'anthraquinone pure recueillie après les opérations de dilution du milieu sulfurique final par de l'acide sulfurique à 70 % en poids pour insolubiliser l'anthraquinone, de filtration et de lavage de l'anthraquinone insolubilisée, est de 79 %.

**Revendications**

1. Procédé de décomposition de complexes acide ortho-benzoyl-benzoïque, fluorure d'hydrogène, trifluorure de bore, de formule générale :

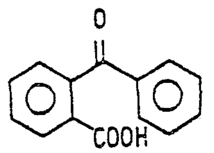 , n HF, m $BF_3$ (I)

dans laquelle n et m sont compris entre 1 et 6, d'une part pour récupérer le fluorure d'hydrogène et le trifluorure de bore, d'autre part pour obtenir l'anthraquinone résultant de la cyclisation de l'acide orthobenzoyl-benzoïque, caractérisé en ce que le complexe est soumis à l'action de l'acide sulfurique de concentration au moins égale à 96 % en poids ou d'un oléum, à une pression au plus égale à la pression atmosphérique et à une température comprise entre 100 °C et 180 °C.

2. Procédé selon la revendication 1, caractérisé en ce que la quantité pondérale d'acide sulfurique est égale à 1 à 3 fois celle de l'acide orthobenzoyl-benzoïque contenu dans le complexe.

3. Procédé selon la revendication 1, caractérisé en ce que la quantité pondérale d'oléum est égale à 0,5 à 2 fois celle de l'acide orthobenzoyl-benzoïque contenu dans le complexe.

4. Procédé selon l'une quelconque des revendications 1 à 3, caractérisé en ce que la durée de l'opération de décomposition du complexe est comprise entre 15 minutes et 2 heures.

5. Procédé selon l'une quelconque des revendications 1 à 4, caractérisé en ce que la décomposition du complexe a lieu en présence d'une quantité d'acide borique suffisante pour transformer en trifluorure de bore tout ou partie du fluorure d'hydrogène contenu dans le complexe.

6. Procédé selon l'une quelconque des revendications 1 à 5, caractérisé en ce que le milieu réactionnel final est dilué par de l'eau pour insolubiliser l'anthraquinone.

7. Procédé selon l'une quelconque des revendications 1 à 5, caractérisé en ce que le milieu réactionnel final est dilué par une solution aqueuse d'acide sulfurique pour insolubiliser l'anthraquinone.

**Claims**

1. Process for the decomposition of complexes of ortho-benzoylbenzoic acid, hydrogen fluoride and boron trifluoride, of general formula :

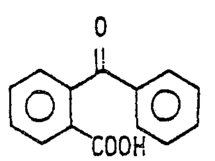 , n HF, m $BF_3$ (I)

in which n and m are from 1 to 6, on the one hand in order to recover the hydrogen fluoride and the boron trifluoride and, on the other hand, in order to obtain the anthraquinone resulting from the cyclization of ortho-benzoylbenzoic acid, characterized in that the complex is subjected to the action of sulphuric acid at a concentration of at least 96 % by weight or of an oleum, at a pressure not exceeding atmospheric pressure and at a temperature of between 100 °C and 180 °C.

2. Process according to claim 1, characterized in that the weight quantity of sulphuric acid is equal to 1 to 3 times that of the ortho-benzoylbenzoic acid present in the complex.

3. Process according to claim 1, characterized in that the weight quantity of oleum is equal to 0.5 to 2 times that of ortho-benzoylbenzoic acid present in the complex.

4. Process according to any one of claims 1 to 3, characterized in that the duration of the complex-decomposition operation is between 15 minutes and 2 hours.

5. Process according to any one of claims 1 to 4, characterized in that the decomposition of the complex takes place in the presence of a quantity of boric acid which is sufficient to convert into boron trifluoride all or part of the hydrogen fluoride present in the complex.

6. Process according to any one of claims 1 to 5, characterized in that the final reaction medium is

diluted with water in order to insolubilize the anthraquinone.

7. Process according to any one of claims 1 to 5, characterized in that the final reaction medium is diluted with an aqueous solution of sulphuric acid in order to insolubilize the anthraquinone.

**Patentansprüche**

1. Verfahren zur Zersetzung der Komplexe aus ortho-Benzoyl-Benzoesäure, Fluorwasserstoff und Bortrifluorid mit der allgemeinen Formel :

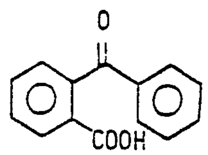 , n HF, m $BF_3$ (I)

in der n und m zwischen 1 und 6 liegen, einerseits zur Rückgewinnung des Fluorwasserstoffs und des Bortrifluorids, andererseits zur Herstellung des Anthrachinons, das bei der Zyklisierung der ortho-Benzoyl-Benzoesäure entsteht, dadurch gekennzeichnet, daß man auf den Komplex Schwefelsäure mit einer Konzentration von mindestens 96 Gew.% oder Oleum einwirken läßt, bei einem Druck der weitgehend gleich dem atmosphärischen Druck ist und bei einer Temperatur, die zwischen 100 °C und 180 °C liegt.

2. Verfahren nach Anspruch 1, dadurch gekennzeichnet, daß die Gewichtsmenge der Schwefelsäure dem 1 bis 3-fachen derjenigen der ortho-Benzoyl-Benzoesäure, die in dem Komplex enthalten ist, entspricht.

3. Verfahren nach Anspruch 1, dadurch gekennzeichnet, daß die Gewichtsmenge des Oleums dem 0,5 bis 2-fachen derjenigen der in dem Komplex enthaltenden ortho-Benzoyl-Benzoesäure entspricht.

4. Verfahren nach einem der Ansprüche 1 bis 3, dadurch gekennzeichnet, daß der Prozeß der Zersetzung des Komplexes zwischen 15 Minuten und 2 Stunden dauert.

5. Verfahren nach einem der Ansprüche 1 bis 4, dadurch gekennzeichnet, daß die Zersetzung des Komplexes in Gegenwart einer Menge Borsäure stattfindet, die ausreichend ist, um einen Teil oder den gesamten im Komplex enthaltenen Fluorwasserstoff in Bortrifluorid umzuwandeln.

6. Verfahren nach einem der Ansprüche 1 bis 5, dadurch gekennzeichnet, daß das am Ende der Reaktion erhaltene Reaktionsmedium mit Wasser verdünnt wird, damit das Anthrachinon unlöslich wird.

7. Verfahren nach einem der Ansprüche 1 bis 5, dadurch gekennzeichnet, daß das am Ende der Reaktion erhaltene Reaktionsmedium mit einer wässrigen Schwefelsäurelösung verdünnt wird, damit das Anthrachinon unlöslich wird.

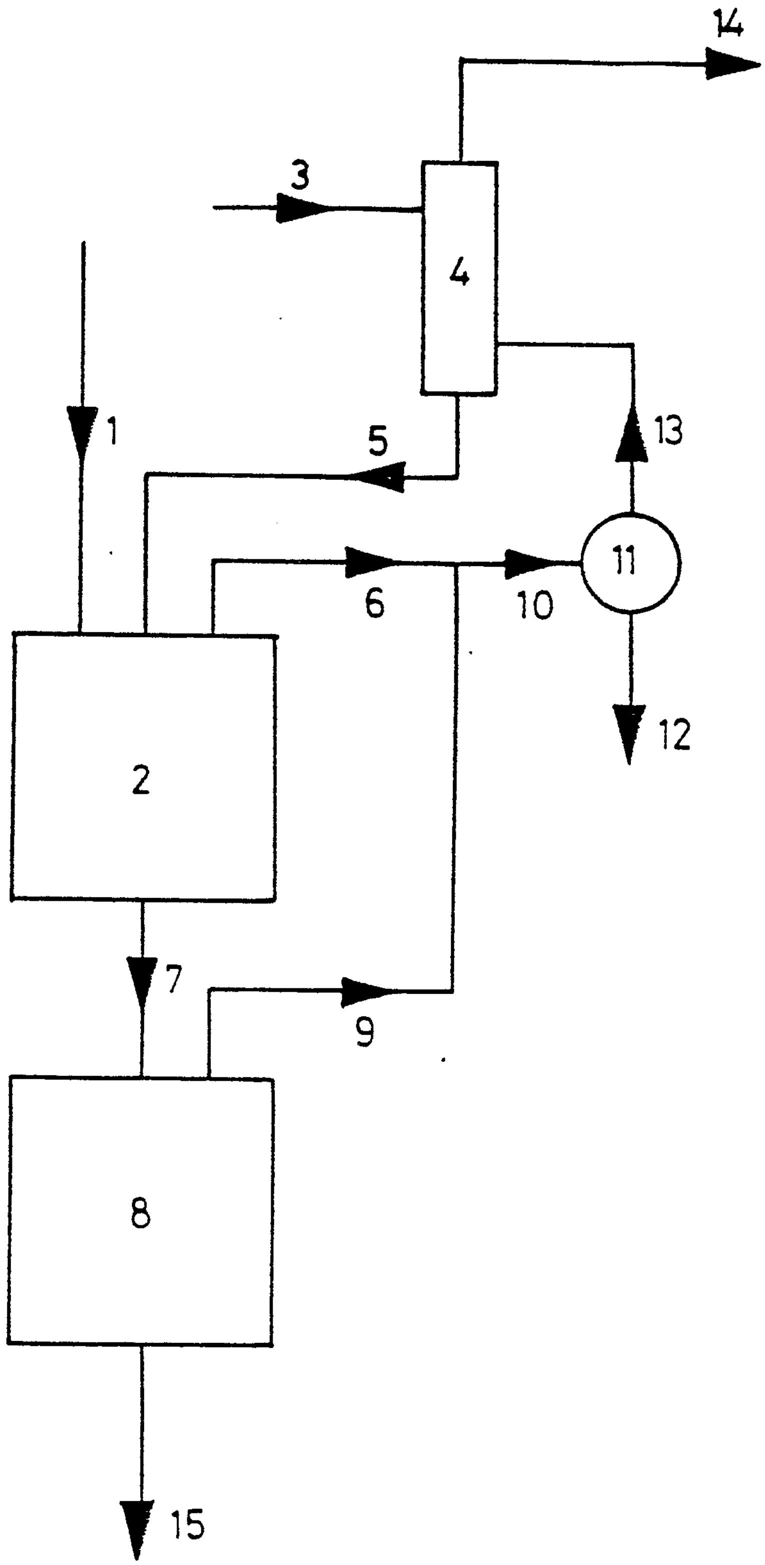
14
3
4
1
5
13
11
6
10
12
2
7
9
8
15